# EUROPEAN PATENT SPECIFICATION

(11) **EP 1 718 357 B1**
(45) Date of publication and mention of the grant of the patent: **13.10.2010**
(21) Application number: 05702942.3
(22) Date of filing: 10.02.2005
(51) Int. Cl.: A61M 25/00, A61M 25/01

(54) **Catheter System with active Position-markers**
Kathetersystem mit aktiven Positionsmarkierungen
Système de catheter avec des repères actives

(30) Priority: 18.02.2004 EP 04100643
(43) Date of publication of application: 08.11.2006
(73) Proprietor: Philips Intellectual Property & Standards GmbH, 20099 Hamburg (DE); Koninklijke Philips Electronics N.V., 5621 BA Eindhoven (NL)
(72) Inventor: BORGERT, Jörn, Weisshausstr. 2, 52066 Aachen (DE); KRÜGER, Sascha, Weisshausstr. 2, 52066 Aachen (DE); TIMINGER, Holger, Weisshausstr. 2, 52066 Aachen (DE)
(86) International application number: PCT/IB2005/050523
(87) International publication number: WO 2005/082441

(56) References cited:
- EP-A- 1 199 031
- WO-A-94/11038
- WO-A-95/18646
- WO-A-97/00043
- US-A- 5 203 338
- US-A- 5 269 793
- US-A1- 2003 060 700
- US-A1- 2003 139 689
- US-A1- 2003 163 037
- US-B1- 6 632 196

## Description

The invention relates to a catheter system comprising a first and a second catheter element, which are coupled to each other and can be used in a method for navigation.

A typical application of catheter systems consists in the exploration and treatment of stenosis (vessel constriction). The success of the medical intervention depends in essence on whether the catheter system can be positioned and navigated with greater precision in relation to the stenosis. As a rule, pre-operative images of the vascular system are used as what are called "vessel maps" on which the current position of the catheter system is shown for the doctor in attendance. Methods have been developed for this purpose, by means of which the self-movements of the vascular system caused by heartbeat and breathing can be largely compensated and which make rough navigation of the catheter to the stenosis possible.

However, the subsequent fine navigation of the catheter at the location of the stenosis remains problematic. A guide wire must be led through the stenosis for this purpose, which guide wire can follow the actual treatment catheter. In this context a catheter system with a tubular outer catheter is known from US 2003/0139689 A1, through the inner channel of which catheter system a guide wire runs. The guide wire can be pushed in advance independently of the outer catheter and has X-ray proof markings at its tip, which markings can be located on an X-ray image. It should be possible to improve the steering of the catheter lead at vessel branching or stenoses by specially configuring the elasticity characteristics of the outer and inner catheter elements. A monitored navigation of the guide wire, however, requires X-ray fluoroscopic observation and possibly injecting contrast agents, involving a related burden on the patient.

Against this background, it was an object of the present invention to provide means for simplified fine navigation for a catheter system in a vascular system.

This object is achieved by means of a catheter system having the features as mentioned in claim 1. Advantageous designs are given in the dependent claims.

The catheter system as invented comprises:
- A first catheter element with at least a first active localizer placed on it, whose spatial position (i.e. position and/or orientation) can be determined by using suitable methods. An "active localizer" in this context is understood to mean a localizer that is not, or not only passively, represented on an image (such as an X-ray-proof marking), but that generates data or signals independently of the imaging method, which make it possible to determine a position. In particular, the active localizer itself can measure or emit signals. Active localizers that are not additionally subject to what are called "line-of-sight restrictions" are also designated as "non-line-of-sight" localizers.
- A second catheter element with at least a second active localizer placed on it, whose spatial position can be determined.

The two catheter elements are coupled mechanically, where the coupling should be designed such that it makes a sliding movement of the catheter elements relative to each other possible. For example, the catheter elements could run next to each other as a string and be coupled to each other through eyebolts or guide bars in such a manner that they can be moved relative to each other only longitudinally. Each of the two catheter elements and in general even the two catheter elements together can be navigated and used together like a single catheter. It should be pointed out that the role of the first and the second catheter element is completely symmetrical, so the features required of a catheter element as given below can also be assumed mutatis mutandis for the other catheter element. Furthermore, it is obvious that the catheter system can analogously also contain more than two coupled catheter elements with active localizers.

The advantage of the described catheter system is that it allows a very precise fine navigation of the individual catheter elements. This is possible, because both catheter elements bear their own active localizers and can moreover be shifted relative to each other. One of the catheter elements can, therefore, be held fast, for example the first one, while only the other catheter element is pushed ahead. The position of the second localizer in relation to the first localizer can be determined with very high precision, as against the absolute spatial position of a localizer, which can typically be measured only with limited accuracy due to organ movements and/or disturbing ambient influences. The first localizer at rest can thus be used as a reference point for a fine navigation of the second localizer or the second catheter element, respectively. Furthermore, the configuration of the markers as active localizers is advantageous, so that the navigation can be effected without the constant use of X-ray radiation.

The mechanical coupling of the catheter elements is preferably realized in that the first catheter element is hollow and has a channel running longitudinally, through which the second catheter element is guided. Typically, in this case, the first catheter element is the actual treatment catheter, which carries, for example, a balloon for widening a stenosis, while the second catheter element is a guide wire. The mechanical construction of such a catheter system can be similar to the one described in US 2003/0139689 A1.

According to another design of the catheter system, this comprises at least one fixing device, with the help of which the first catheter element or the second catheter element can be fixed, in relation to a surrounding vessel. Fixing ensures that the catheter element concerned cannot move in relation to the vascular system, so that it serves as a reference for the observation of the movement of the other catheter element relative to the vascular system.

There are different options for the realization of the active localizers. According to a first alternative, at least one of the localizers can be a magnetic field sensor that can measure the strength and direction of an external (spatially and/or temporally inhomogeneous) magnetic field. The external magnetic field is generated here typically by a field generator near the examined patient and a conclusion about its spatial position can be drawn from the measurement data of the magnetic field sensor. In principle, the problem with such a "magnetic tracking" is that the magnetic field and thereby the accuracy of measurement suffers from interference from external factors such as metallic bodies etc. If, however, both active localizers of the catheter system are configured as magnetic field sensors, such interference affects both the sensors in equal measure, so they balance each other in the determination of the relative positions, up to interference of higher and therefore negligible orders.

According to another embodiment, at least one of the localizers contains a source for electromagnetic and/or acoustic radiation, where the position of this source can be located through external sensors. In particular a localizer can be an emitter for electromagnetic radiation from the near infrared (NIR) or an ultrasonic emitter. The position of such a radiation source can be determined, for example, by using stereoscopic methods, in which the intersection point of the lines of sight of the radiation source is determined from different spatial points.

The localizers on both catheter elements are preferably so placed that they lie at a distance of maximum 10 cm, particularly preferably 5 cm from each other in the typical usage of the catheter system. This way it is ensured that the distance between the localizers does not exceed the top limit, so that they are exposed to external influences to an almost equal extent. When measuring the relative positions of the two localizers, therefore, the corresponding influences balance each other, reducing them to an insignificant factor.

The invention may be used in a method for navigation of a catheter system in a vascular system, where the catheter system comprises a first and a second catheter element, which are coupled to each other so as to afford a sliding movement and each of them bears at least a first or second active localizer respectively. The catheter system can particularly be a catheter system of the kind mentioned above. The method comprises the following steps of:
a) determining the spatial position of the first localizer on the first catheter element relative to the vascular system.
b) determining the spatial position of the second localizer on the second catheter element relative to the first localizer. In this step, the associated methods for position determination of the respective localizers can be used, where the relative position of the second localizer is obtained from the difference in the determined positions.

The advantage of the method is that it makes it possible to provide very precise fine navigation of the second catheter element in a vascular system (it being again pointed out here that, in principle, the role of the first and the second catheter element is symmetrical). The reason for this is that, during the navigation, the position of the second catheter element is determined relative to the first catheter element, which is possible with comparatively great accuracy. Effects from organ movements or external interference are experienced more or less equally by both localizers, so they are balanced in the relative position.

In order to improve the accuracy of the method, the first catheter element is preferably fixed in relation to the vascular system. During a movement of the second catheter element, its position can then be measured in relation to the first localizer, where the fixing ensures that the first localizer simultaneously represents a defined reference point in relation to the vascular system.

In step a) of the method, the position of the first localizer must be determined in relation to the vascular system. This is possible particularly by generating an image of the vascular system with the catheter system contained in it and subsequently determining the spatial position of the first localizer relative to the vascular system on this image. Said image can, for example, be an X-ray image or a magnetic resonance image. It should be ensured here that the position of the first localizer can be determined (passively) with sufficient accuracy on the image, for example by providing the first localizer with an X-ray-proof material. Another possibility is to register the previously recorded vascular map by a suitable method i.e. determine the mutual correlation between the coordinate systems of the vascular map and of the active localizer or of the real vascular tree. Furthermore, it should be ensured during magnetic resonance imaging that the catheter elements are compatible with it and do not contain, for example, any components which could lead to dangerous heating during the recording.

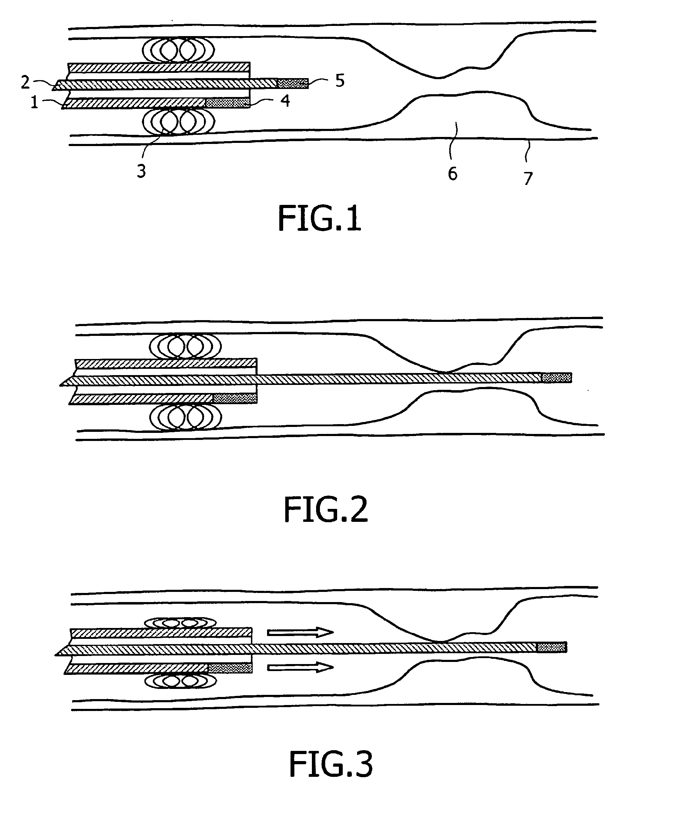

The invention is elucidated below with the help of exemplary figures. Figs 1 to 6 show schematically successive steps during the navigation of a catheter system as invented in the region of a stenosis.

Fig. 1 shows the tip of a catheter system as invented in a vessel 7, where the vessel has a stenosis 6 due to deposits on the inner vessel walls. The catheter system comprises a first catheter element 1 (called "catheter" below for short), which has a channel inside, running longitudinally, through which a second catheter element is guided in the form of a guide wire 2. The guide wire 2 may be moved as desired relative to the outer catheter 1 in longitudinal direction.

Furthermore, the outer catheter 1 as well as the guide wire 2 bear in each case an active localizer 4 or 5 respectively on their tips. This may be, for example, a magnetic field probe of a magnetic tracking system. The spatial position of the catheter elements in relation to an external coordinate system can be determined by means of the localizers 4, 5 with comparatively good accuracy. This current spatial position is shown, as a rule, on a preoperatively generated 3-dimentional vessel map for navigation of the catheter system. The methods so far known typically deliver an accuracy of 2-3 mm for compensation of organ movements, so a rough navigation of the catheter system up to the stenosis 6 is already possible. This, however, makes subsequent fine navigation of the catheter system through the stenosis 6 more problematic. Efforts are made here to find a way through the stenosis 6 using the guide wire 2, where one has to proceed very carefully due to the hazard of loosening of the deposits. In many cases, the navigation of the guide wire through the stenosis 6 is the most time-consuming part of an intervention, which can take up to 20 minutes. This problem should be solved by the proposed catheter system and the method possible with it.

As already elucidated, the outer catheter 1 as well as the guide wire 2 bear in each case their own active localizer 4 or 5 respectively. Furthermore, it can be seen in Fig. 1 that the outer catheter 1 has an (optional) fixing device 3, by means of which its position relative to the vessel 7 can be fixed. For example, the device 3 can contain a braided grid, which can be expanded radially to clamp the outer catheter 1 firmly in the vessel 7. In this way it is ensured that the catheter 1 does not make any movement relative to the vessel 7, but identically follows its movement.

After fixing the outer catheter 1 in the vessel, an X-ray-fluoroscopic image of the vascular system can be generated where preferably a contrast agent is injected simultaneously for highlighting the path of the vessel. The position of the catheter 1 or of a linked X-ray-proof marker (e.g. of the localizer 4) can then be determined on this X-ray image, so that the fixed position of the localizer 4 relative to the catheter system 1 is known for the subsequent steps of the method. This information can also be gleaned by registering a previously recorded vascular map by a suitable method i.e. by establishing the mutual correlation between the coordinate systems of the vessel map and of the active localizer or of the real vascular tree.

In the next step (Fig. 2), the inner guide wire 2 is pushed ahead through the stenosis 6, where the spatial positions of the magnetic localizers 4 and 5 are (actively) measured continuously. The difference between these two positions provides the position of the localizer 5 on the guide wire 2 relative to the vascular system, because the localizer 4 on catheter 1 is fixed relative to the vascular system. This measurement of the relative position has great accuracy, because both localizers 4, 5 are affected almost equally by the movements of the vascular system due to their close proximity, the differences in the localizers thus balancing each other. The same is applicable for interference due to metals in the vicinity (e.g. the C-arc of X-ray equipment) or the like. The movement of the guide wire 2 can therefore be followed with great precision, for example, on a static vessel map. This makes it possible to navigate the guide wire 2 very much faster through the stenosis 6 than by the conventional method, until it reaches the end position on the other side of the stenosis 6 as shown in Fig. 2.

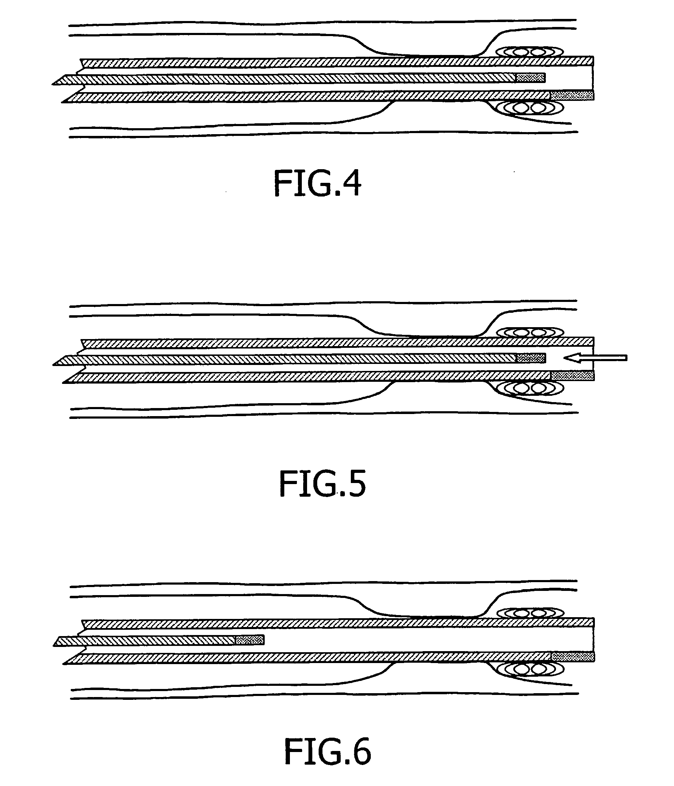

After passing the stenosis 6, the fixing of the outer catheter 1 is released and it is then pushed through the stenosis 6 following the guide wire 2 (Figs. 3, 4).

Subsequently, the guide wire 2 can be retracted in the outer catheter 1 up to a position shortly before the stenosis 6 (Figs. 5, 6).

The catheter system as invented and the navigation method associated to it thus make a very precise fine navigation possible, which is particularly helpful in the area of stenoses. The core concept here is to provide two catheter elements movable relative to each other, each with its own active localizer, through which the relative movement of the catheter elements can be measured with high accuracy, because the interference factors (movement, fields etc) are balanced. Furthermore, one of the catheter elements can be fixed to the surroundings, so that the movement of the other catheter element relative to these surroundings can be observed. In this manner, iterative navigation with high precision is possible. Other advantages of the method consist in the fact that catheter navigation is possible with comparatively low X-ray burden. Moreover, the method supports techniques based on (especially two) localizers, for example the selection of a region of interest with the help of the localizers and method for movement compensation, which are based on the local reference sensors and movement models.

Even though the medical application of the catheter system for navigation through a stenosis is shown in the figures, the present invention is in no way restricted to such applications, but can be used in all cases with comparable problems.

## Claims

1. A catheter system, comprising:
- a first catheter element (1) with at least a first active localizer (4) placed on it, whose spatial position can be determined;
- a second catheter element (2) with at least a second active localizer (5) placed on it, whose spatial position can be determined;
wherein the first and the second catheter element are coupled in such a manner that a sliding movement relative to each other is possible.

2. A catheter system as claimed in claim 1, **characterized in that** the first catheter element (1) has a channel running in longitudinal direction, through which the second catheter element (2) is guided.

3. A catheter system as claimed in claim 1, **characterized in that** it comprises a fixing device (3), by means of which at least one of the catheter elements (1) can be fixed in a surrounding vessel (7).

4. A catheter system as claimed in claim 1, **characterized in that** at least one of the localizers (4, 5) is a magnetic field sensor in an external magnetic field for determining the position.

5. A catheter system as claimed in claim 1, **characterized in that** at least one of the localizers contains a source for electromagnetic and/or acoustic radiation.

6. A catheter system as claimed in claim 1, **characterized in that** the localizers (4, 5) are arranged such that they are at a distance of less than 10 cm, preferably less than 5 cm from each other during the use of the catheter system.

## Patentansprüche

1. Kathetersystem, das Folgendes umfasst:
- ein erstes Katheterelement (1) mit mindestens einem darauf angeordneten ersten aktiven Lokalisierer (4), dessen räumliche Position ermittelt werden kann;
- ein zweites Katheterelement (2) mit mindestens einem darauf angeordneten zweiten aktiven Lokalisierer (5), dessen räumliche Position ermittelt werden kann;
wobei das erste und das zweite Katheterelement auf derartige Weise gekoppelt sind, dass eine gleitende Bewegung relativ zueinander möglich ist.

2. Kathetersystem nach Anspruch 1, **dadurch gekennzeichnet, dass** das erste Katheterelement (1) einen in Längsrichtung verlaufenden Kanal hat, durch den das zweite Katheterelement (2) geführt wird.

3. Kathetersystem nach Anspruch 1, **dadurch gekennzeichnet, dass** es eine Fixiervorrichtung (3) umfasst, mit deren Hilfe mindestens eines der Katheterelemente in einem umgebenden Gefäß (7) fixiert werden kann.

4. Kathetersystem nach Anspruch 1, **dadurch gekennzeichnet, dass** mindestens einer der Lokalisierer (4, 5) ein Magnetfeldsensor in einem externen Magnetfeld zum Ermitteln der Position ist.

5. Kathetersystem nach Anspruch 1, **dadurch gekennzeichnet, dass** mindestens einer der Lokalisierer eine Quelle elektromagnetischer und/oder akustischer Strahlung umfasst.

6. Kathetersystem nach Anspruch 1, **dadurch gekennzeichnet, dass** die Lokalisierer (4, 5) so angeordnet sind, dass sie sich während der Verwendung des Kathetersystems in einem Abstand von weniger als 10 cm, vorzugsweise von weniger als 5 cm, voneinander befinden.

## Revendications

1. Système de cathéter, comprenant :
- un premier élément de cathéter (1) avec au moins un premier localisateur actif (4) placé sur celui-ci, dont la position spatiale peut être déterminée ;
- un deuxième élément de cathéter (2) avec au moins un deuxième localisateur actif (5) placé sur celui-ci, dont la position spatiale peut être déterminée ;
dans lequel le premier et le deuxième élément de cathéter sont couplés de telle manière qu'un mouvement coulissant soit possible fun par rapport à l'autre.

2. Système de cathéter selon la revendication 1, **caractérisé en ce que** le premier élément de cathéter (1) comporte un canal disposé dans la direction longitudinale, à travers lequel le deuxième élément de cathéter (2) est guidé.

3. Système de cathéter selon la revendication 1, **caractérisé en ce qu'**il comprend un dispositif de fixation (3), au moyen duquel au moins l'un des éléments de cathéter (1) peut être fixé dans un récipient (7) environnant.

4. Système de cathéter selon la revendication 1, **caractérisé en ce qu'**au moins l'un des localisateurs (4, 5) est un capteur de champ magnétique dans un champ magnétique externe pour déterminer la position.

5. Système de cathéter selon la revendication 1, **caractérisé en ce qu'**au moins l'un des localisateurs contient une source de rayonnement électromagnétique et/ou acoustique.

6. Système de cathéter selon la revendication 1, **caractérisé en ce que** les localisateurs (4, 5) sont disposés de telle sorte qu'ils se trouvent à une distance de moins de 10 cm, de préférence moins de 5 cm l'un de l'autre pendant l'utilisation du système de cathéter.
